(19) Europäisches Patentamt
European Patent Office
Office européen des brevets

(11) Veröffentlichungsnummer: **0 134 515**
**A1**

(12) # EUROPÄISCHE PATENTANMELDUNG

(21) Anmeldenummer: **84108384.3**

(22) Anmeldetag: **17.07.84**

(51) Int. Cl.⁴: **C 07 D 249/08**
C 07 D 233/61, A 01 N 43/50
A 01 N 43/653

(30) Priorität: **10.08.83 DE 3329213**

(43) Veröffentlichungstag der Anmeldung:
**20.03.85 Patentblatt 85/12**

(84) Benannte Vertragsstaaten:
**BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Skötsch, Carlo, Dr.**
**Kaiserin-Augusta-Allee 83**
**D-1000 Berlin 10(DE)**

(72) Erfinder: **Baumert, Dietrich, Dr.**
**Schulzendorferstrasse 108 B**
**D-1000 Berlin 28(DE)**

(72) Erfinder: **Krähmer, Hansjörg, Dr.**
**Fürstendamm 10 a**
**D-1000 Berlin 28(DE)**

(54) **Azolyl-Propannitrile, Verfahren zur Herstellung dieser Verbindungen sowie diese enthaltende biozide Mittel.**

(57) Die Erfindung betrifft Azolyl-Propannitrile der allgemeinen Formel

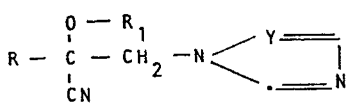

I

in der

R einen gegebenenfalls ein- oder mehrfach, gleich oder verschieden durch Halogen, ($C_1$-$C_4$)-Alkyl, ($C_1$-$C_4$)-Alkoxy, Trifluormethyl, Cyano oder die Nitrogruppe substituierten aromatischen Kohlenwasserstoffrest,

$R_1$ einen gegebenenfalls durch $C_1$-$C_3$-Alkyl substituierten $C_3$-$C_8$-Cycloalkylrest und

Y N oder CH bedeuten und deren Säureadditionssalze mit anorganischen und organischen Säuren, Verfahren zur Herstellung dieser Verbindungen sowie diese enthaltende biozide Mittel insbesondere mit fungizider Wirkung. Außerdem weisen die Mittel eine wuchsregulatorische sowie eine bakterizide Wirkung auf.

EP 0 134 515 A1

Croydon Printing Company Ltd.

0134515

Die Erfindung betrifft neue Azolyl-propannitrile, Verfahren zur Herstellung dieser Verbindungen sowie diese enthaltende biozide Mittel insbesondere mit fungizider Wirkung.

Imidazolyl-propionitrilderivate mit fungizider Wirkung sind bereits bekannt (DE-OS 26 04 047). Diese weisen jedoch ein relativ enges Wirkungsspektrum auf, was unbefriedigend ist.

Imidazolyl-Propionitrile mit biozider Wirkung sind ebenfalls bereits bekannt (DE-OS 31 25 780). Trotz deren guter Wirksamkeit besteht weiterhin ein Bedürfnis nach Verbindungen mit noch besseren Eigenschaften.

Aufgabe der vorliegenden Erfindung ist es, neue Propionitrilderivate zu entwickeln, welche breit gefächerte Anwendungsmöglichkeiten insbesondere auf dem Gebiet des Pflanzenschutzes eröffnen und überlegene fungizide Wirkungen entfalten.

Diese Aufgabe wird erfindungsgemäß gelöst durch Azolyl-Propannitrile der allgemeinen Formel

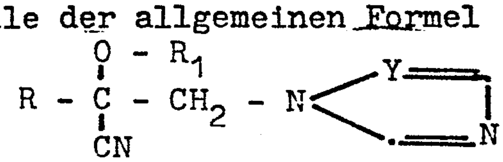

I

in der

R einen gegebenenfalls ein- oder mehrfach, gleich oder verschieden durch Halogen, $(C_1-C_4)$-Alkyl, $(C_1-C_4)$-Alkoxy, Trifluormethyl, Cyano oder die Nitrogruppe substituierten aromatischen Kohlenwasserstoffrest,

$R_1$ einen gegebenenfalls durch $C_1-C_3$-Alkyl substituierten $C_3-C_8$-Cycloalkylrest und

- 2 -

Y N oder CH bedeuten und deren Säureadditionssalze mit anorganischen und organischen Säuren.

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00
54 FH IV 35718
Formular-Nr.: 1439-2

Die erfindungsgemäßen Verbindungen sind im weitesten Sinne biozid wirksam, zeichnen sich jedoch insbesondere durch eine fungizide Wirkung aus, worin sie überraschenderweise bekannte Wirkstoffe analoger Konstitution und Wirkungsrichtung übertreffen.

Die fungizide Wirkung erstreckt sich überraschenderweise gegen Pilze unterschiedlichster systematischer Stellung. Bei der Behandlung oberirdischer Pflanzenteile schützen sie gegen windbürtige Krankheitserreger. Gegen samenübertragbare Krankheitserreger kann man die Verbindungen zur Saatgutbehandlung einsetzen. Außerdem wirken diese systemisch, das heißt, sie werden von den Wurzeln der Pflanzen zum Beispiel nach Einbringung bei der Saat aufgenommen, werden in die oberirdischen Teile der Pflanze transportiert und schützen diese gegen Krankheitserreger.

Als weitere Wirkungen sind zu nennen eine wuchsregulatorische und eine bakterizide Wirkung.

Wegen des erkannten breiten Wirkungsspektrums eignen sich die Verbindungen nicht nur zum Schutz von Kulturpflanzen, sondern auch zum Materialschutz und zur Bekämpfung humanpathogener und tierpathogener Mikroben, woraus sich breit gefächerte Anwendungsmöglichkeiten ergeben.

Je nach der speziellen Bedeutung der Substituenten ergeben sich hierbei schwerpunktmäßige Anwendungsgebiete, bei denen die Verbindungen herausragende Wirkung entfalten. So können diese jeweils als Fungizide, Pflanzenwachstumsregulatoren oder Bakterizide eingesetzt werden.

- 3 -

Gewe.blicher Rechts...

0134515

Die erfindungsgemäßen Verbindungen finden als Pflanzenwachstumsregulatoren Verwendung. Pflanzenwachstumsregulatoren zeichnen sich zum Beispiel durch folgende Anwendungsmöglichkeiten aus:

Hemmung des vegetativen Wachstums bei holzigen und krautigen Pflanzen zum Beispiel an Straßenrändern, Gleisanlagen u.a., um ein üppiges Wachstum zu unterbinden. Wuchshemmung beim Getreide, um das Lagern oder Umknicken zu unterbinden, bei Baumwolle zur Ertragserhöhung.

Beeinflussung der Verzweigung von vegetativen und generativen Organen bei Zier- oder Kulturpflanzen zur Vermehrung des Blütenansatzes oder bei Tabak und Tomate zur Hemmung von Seitentrieben.

Verbesserung der Fruchtqualität, zum Beispiel eine Zuckergehaltssteigerung beim Zuckerrohr, bei Zuckerrüben oder bei Obst, und eine gleichmäßigere Reife des Erntegutes, die zu höheren Erträgen führt.

Erhöhung der Widerstandskraft gegen klimatische Einflüsse wie Kälte und Trockenheit.

Beeinflussung des Latexflusses bei Gummipflanzen.

Ausbildung parthenokarper Früchte, Pollensterilität und Geschlechtsbeeinflussung sind ebenfalls Anwendungsmöglichkeiten.

Kontrolle der Keimung von Samen oder des Austriebs von Knospen.

Entlaubung oder Beeinflussung des Fruchtfalls zur Ernteerleichterung.

- 4 -

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

Die erfindungsgemäßen Verbindungen eignen sich insbesondere zur Beeinflussung des Wachstums von Beta-Rüben, zeigen aber auch bei Getreide, Soja und Baumwolle gewünschte wuchsregulatorische Effekte.

Die Aufwandmengen betragen je nach Anwendungsziel im allgemeinen von 0,05 bis 5 kg Wirkstoff/ha, gegebenenfalls können auch höhere Aufwandmengen eingesetzt werden.

Die Anwendungszeit richtet sich nach dem Anwendungsziel und den klimatischen Bedingungen.

In den durch die allgemeine Formel I gdennzeichneten Verbindungen können zum Beispiel bedeuten:

R 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 3-Jodphenyl, 4-Jodphenyl, 2,4-Dichlorphenyl, 3,4-Dichlorphenyl, 2,6-Dichlorphenyl, 2-Methylphenyl, 3-Methylphenyl, 4-Methylphenyl, 2-Äthylphenyl, 3-Ätyhlphenyl, 4-Äthylphenyl, 2-Propylphenyl, 3-Propylphenyl, 4-Propylphenyl, 2-Isopropylphenyl, 3-Isopropylphenyl, 4-Isopropylphenyl, 2-Butylphenyl, 3-Butylphenyl, 4-Butylphenyl, 2-sek.-Butylphenyl, 3-sek.-Butylphenyl, 4-sek.-Butylphenyl, 2-tert.-Butylphenyl, 3-tert.-Butylphenyl, 4-tert.-Butylphenyl, 2-Methoxyphenyl, 3-Methoxyphenyl, 4-Methoxyphenyl, 2-Äthoxyphenyl, 3-Äthoxyphenyl, 4-Äthoxyphenyl, 2-Methylthiophenyl, 3-Methylthiophenyl, 4-Methylthiophenyl, 2-Äthylthiophenyl, 3-Äthylthiophenyl, 4-Äthylthiophenyl,

- 5 -

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 4
Berliner Disconto-Bank AG, Berlin, Konto-Nr. 241/5008, Bankleitzahl 100 70
Berliner Handels-Gesellschaft – Frankfurter Bank –, Berlin,
Konto-Nr. 14-362, Bankleitzahl 100 202 00

54 FH IV 3

2-Trifluormethylphenyl, 3-Trifluormethylphenyl, 4-Trifluormethylphenyl, 2-Nitrophenyl, 3-Nitrophenyl, 4-Nitrophenyl, 3-Fluor-4-methoxyphenyl, 3-Chlor-5-nitrophenyl, 4-Chlor-2-fluorphenyl, 3,4,5-Trimethoxyphenyl oder 5-Chlor-2-nitrophenyl,

$R_1$ Cyclopropyl, Cyclobutyl, Cyclopentyl, Cyclohexyl, Cycloheptyl, Cyclooctyl, 2-Methylcyclopentyl, 2-Methylcyclohexyl, 2,2-Dimethylcyclopentyl oder 2,2-Dimethylcyclohexyl und

Y N oder CH bedeuten.

Zur Bildung der Säureadditionssalze kommen sowohl anorganische wie auch organische Säuren in Frage. Als Beispiel seien genannt: die Chlorwasserstoffsäure und die Bromwasserstoffsäure, außerdem Phosphorsäure, Schwefelsäure, insbesondere Salpetersäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure.

Die Säureadditionssalze können nach den üblichen Salzbildungsverfahren, zum Beispiel durch Lösen einer Verbindung der Formel I in einem geeigneten Lösungsmittel und Hinzufügen der Säure erhalten werden.

Eine herausragende fungizide Wirkung zeigen beispielsweise die folgenden Verbindungen:



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin, Konto-Nr. 14-362, Bankleitzahl 100 202 00

54 FH IV 35718

2-(4-Chlorphenyl)-2-cyclopentyloxy-3-(imidazol-1-yl)-propannitril
Hydronitrat
2-(4-Chlorphenyl)-2-cyclopentyloxy-3-(1,2,4-triazol-1-yl)-propannitril
Hydronitrat
2-Cyclopentyloxy-3-(imidazol-1-yl)-2-(2-methyl-phenyl)-propannitril
Hydronitrat
2-Cyclopentyloxy-2-phenyl-3-(1,2,4-triazol-1-yl)-propannitril
Hydronitrat
2-(2-Chlorphenyl)-2-cyclopentyloxy-3-(1,2,4-triazol-1-yl)-propannitril
Hydronitrat
2-Cyclopentyloxy-3-(imidazol-1-yl)-2-phenyl-propannitril
Hydronitrat

Außer den vorbezeichneten Wirkungen entfalten die erfindungsgemäßen Verbindungen auch eine bakterizide Wirkung, die weitere Anwendungsmöglichkeiten erlaubt.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen, beziehungs-

- 7

weise Verdünnungsmitteln und gegebenenfalls Netz-, Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxyd, Dimethylformamid, weiterhin Mineralölfraktionen und Pflanzenöle.

Als feste Trägerstoffe eignen sich Mineralerden, zum Beispiel Tonsil, Silicagel, Talkum, Kaolin, Attapulgit, Kalkastein, Kieselsäure und pflanzliche Produkte, zum Beispiel Mehle.

An oberflächenaktiven Stoffen sind zu nennen:
zum Beispiel Calciumligninsulfonat, Polyoxyäthylenalkylphenyläther, Naphthalinsulfonsäuren und deren Salze, Formaldehydkondensate, Fettalkoholsulfate sowie substituierte Benzolsulfonsäuren und deren Salze.

Sofern die Wirkstoffe zur Saatgutbeizung Verwendung finden sollen, können auch Farbstoffe zugemischt werden, um dem gebeizten Saatgut eine deutlich sichtbare Färbung zu geben.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozente flüssige oder feste Trägerstoffe sowie gegebenenfalls bis zu 20 Gewichtsprozente oberflächenaktive Stoffe.

Die Ausbringung der Mittel kann in üblicher Weise erfolgen, zum Beispiel mit Wasser als Träger in Spritzbrühmengen von



Formular-Nr.: 1459-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

etwa 100 bis 10000 Liter/ha. Eine Anwendung der Mittel im sogenannten "Low-Volume-" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. Spritzpulver

a) 40 Gewichtsprozent Wirkstoff
25 Gewichtsprozent Tonmineralien
20 Gewichtsprozent Kieselsäure
10 Gewichtsprozent Zellpech
5 Gewichtsprozent oberflächenaktive Stoffe auf der Basis einer Mischung des Calciumsalzes der Ligninsulfonsäure mit Alkylphenolpolyglykoläthern

b) 20 Gewichtsprozent Wirkstoff
60 Gewichtsprozent Kaolin
15 Gewichtsprozent Kieselsäure
5 Gewichtsprozent oberflächenaktive Stoffe auf Basis des Natriumsalzes des N-Methyl-N-oleyl-taurins und des Calciumsalzes der Ligninsulfonsäure

c) 10 Gewichtsprozent Wirkstoff
60 Gewichtsprozent Tonmineralien
15 Gewichtsprozent Kieselsäure
10 Gewichtsprozent Zellpech
5 Gewichtsprozent oberflächenaktive Stoffe auf Basis des Natriumsalzes des N-Methyl-N-oleyltaurins und des Calciumsalzes der Ligninsulfonsäure



Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl 1
Berliner Handels-Gesellschaft – Frankfurter Bank –, Berlin,
Konto-Nr. 14-362, Bankleitzahl 100 202 00

54 Ft

B. Paste

45 Gewichtsprozent Wirkstoff
5 Gewichtsprozent Natriumaluminiumsilikat
15 Gewichtsprozent Cetylpolyglykoläther mit 8 Mol Äthylenoxid
2 Gewichtsprozent Spindelöl
10 Gewichtsprozent Polyäthylenglycol
23 Teile Wasser

C. Emulsionskonzentrat

25 Gewichtsprozent Wirkstoff
15 Gewichtsprozent Cyclohexanon
55 Gewichtsprozent Xylol
5 Gewichtsprozent Mischung von Nonylphenylpolyoxyäthylen oder Calciumdodecylbenzolsulfonat.

Die neuen erfindungsgemäßen Verbindungen lassen sich zum Beispiel herstellen, indem man Verbindungen der allgemeinen Formel

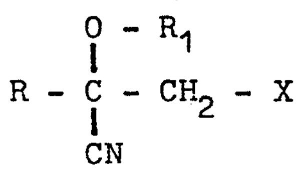

mit Verbindungen der allgemeinen Formel

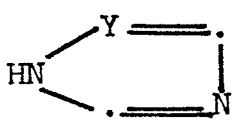

oder deren Alkalisalzen in Gegenwart eines Lösungsmittels und gegebenenfalls in Gegenwart einer Base umsetzt, wobei R, $R_1$ und Y die oben genannte Bedeutung haben und X Halogen oder einen gegebenenfalls in der Seitenkette halogenierten Alkylsulfonyloxy-Rest oder Arylsulfonyloxy-Rest darstellt.



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

Gewerblicher Rechtsschutz

0134515

Als Halogen ist beispielsweise zu nennen: Chlor, Brom oder Jod; als Alkylsulfonyloxy-Reste eignen sich Methyl, Äthyl, Propyl- und die Trifluormethylsulfonylgruppe und als Arylsulfonyloxy-Reste seien beispielsweise genannt die Benzolsulfonyloxy- und p-Toluolsulfonyloxy-Gruppen.

Verwendet man Lösungsmittel, so eignen sich gegenüber den Reaktanden inerte Stoffe vorzugsweise polaren, aprotischen Charakters wie N,N-Dimethylformamid, N-Methylpyrrolidon, Tetramethylharnstoff, Hexamethylphosphorsäuretriamid und Dimethylsulfoxid, aber auch höher siedende aromatische und aliphatische Kohlenwasserstoffe wie Toluol, Chlorbenzol oder Xylol. Die Reaktionstemperatur kann in weiten Grenzen variiert werden. Bevorzugt wird eine Temperatur zwischen 100° C und 200° C. Die Umsetzungen erfolgen unter Normal- oder Überdruck.

Die folgenden Beispiele erläutern die Herstellung der erfindungsgemäßen Verbindungen:



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 1
Berliner Disconto-Bank AG, Berlin, Konto-Nr. 241/5008, Bankleitzahl 100
Berliner Handels-Gesellschaft – Frankfurter Bank –, Berlin,
Konto-Nr. 14-362, Bankleitzahl 100 202 00

54 FH I

B e i s p i e l 1

2-Cyclopentyloxy-3-(imidazol-1-yl)-2-(2-methylphenyl)-propannitril, Hydronitrat

60,7 g (0,188 Mol) 2-Cyclopentyloxy-2-(2-methylphenyl)-3-methylsulfonyloxy-propannitril, 63,9 g (0,938 Mol) Imidazol und 3,88 ml DMF werden 6h bei 160°C Badtemperatur gerührt. Man gießt auf 200 ml Wasser, extrahiert zweimal mit je 200 ml Methylenchlorid und trocknet die organische Phase über $MgSO_4$. Man rotiert im Vakuum ein, löst das Öl in 100 ml Isopropanol und versetzt tropfenweise unter Eiskühlung mit 10 ml 100 %iger Salpetersäure. Man rührt noch 20 min bei RT nach, gibt 200 ml Essigester zu und saugt ab. Man erhält 30,7 g der Titelverbindung vom Fp.: 160°C (Zersetzung)



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

SCHERING AG
Gewerblicher Rechtsschutz
0134515

## Beispiel 2

**2-Cyclopentyloxy-3-(imidazol-1-yl)-2-(2-methylphenyl)-propannitril**

28,5 g des unter Beispiel 1 erhaltenen Hydronitrates werden in 300 ml Methanol gelöst. Innerhalb von 5 min werden bei 5° - 15° C 100 ml verdünnte Ammoniaklösung zugetropft. Man verdünnt mit 750 ml Wasser und extrahiert 2 Mal mit je 300 ml Essigester. Man trocknet über $MgSO_4$, rotiert zur Trockne ein, versetzt das zurückbleibende Öl mit 200 ml Hexan und läßt kristallisieren. Man erhält 21,7 g Titelverbindung vom Fp.: 92°C.

- 13

Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl 1
Berliner Handels-Gesellschaft – Frankfurter Bank –, Berlin,
Konto-Nr. 14-362, Bankleitzahl 100 202 00

54 Ff

Beispiel 3

2-Cyclohexyloxy-2-(2-methylphenyl)-3-(1,2,4-triazol-1-yl)-propannitril, Hydronitrat

9,8 g 1,2,4-Triazol werden in einem Gemisch aus 120 ml Dimethylsulfoxyd und 70 ml Xylol mit 5,7 g NaOH-Plätzchen 2h bei 180°C Badtemperatur erhitzt. Reaktionswasser wird an einem Wasserabscheider abgefangen. Man läßt die Badtemperatur auf 150°C sinken und gibt 32 g 2-Cyclohexyloxy-3-methansulfonyl-2-(2-methylphenyl)-propannitril in wenig DMSO zu. Man rührt 3h bei 150°C. Man gibt auf Eiswasser, extrahiert zweimal mit je 200 ml Methylenchlorid und wäscht die organische Phase nochmal mit 100 ml $H_2O$. Man trocknet über $MgSO_4$, rotiert ein, nimmt in 70 ml Isopropanol auf und versetzt unter Eiskühlung tropfenweise mit 3,9 ml 100 %iger Salpetersäure. Man versetzt mit 50 ml Äther, saugt ab und kristallisiert aus Isopropanol um. Man erhält 14,36 g der Titelverbindung vom Fp.: 194-197°C (Zersetzung).

In analoger Weise lassen sich die folgenden erfindungsgemäßen Verbindungen herstellen:



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

| Beispiel | Name der Verbindungen | Physikalische Konstante |
|---|---|---|
| 4 | 2-Cyclopentyloxy-2-(2-methyl-phenyl)-3-(1,2,4-triazol-1-yl)-propannitril, Hydronitrat | Fp.: 142-144°C (Z) |
| 5 | 2-Cyclohexyloxy-3-(imidazol-1-yl)-2-(2-methylphenyl)-propannitril, Hydronitrat | Fp.: 150-152°C (Z) |
| 6 | 2-Cyclohexyloxy-3-(imidazol-1-yl)-2-(2-methylphenyl)-propannitril | Fp.: 114-116°C |
| 7 | 2-Cyclohexyloxy-2-(2-methyl-phenyl)-3-(1,2,4-triazol-1-yl)-propannitril | Fp.: 82- 85°C |
| 8 | 2-Cyclohexyloxy-3-(imidazol-1-yl)-2-phenyl-propannitril | Fp.: 75°C |
| 9 | 2-Cyclohexyloxy-3-(imidazol-1-yl)-2-phenyl-propannitril, Hydronitrat | Fp.: 211-212°C (Z) |
| 10 | 2-(2-Chlorphenyl)-2-cyclohexyloxy-3-(imidazol-1-yl)-propannitril | Fp.: 128°C |
| 11 | 2-(2-Chlorphenyl)-2-cyclohexyloxy-3-(imidazol-1-yl)-propannitril, Hydronitrat | Fp.: 186-188°C (Z) |
| 12 | 2-Cyclohexyloxy-2-phenyl-3-(1,2,4-triazol-1-yl)-propannitril | Fp.: 94- 97°C |
| 13 | 2-Cyclohexyloxy-2-phenyl-3-(1,2,4-triazol-1-yl)-propannitril, Hydronitrat | Fp.: 185-186°C (Z) |
| 14 | 2-(4-Chlorphenyl)-2-cyclohexyloxy-3-(imidazol-1-yl)-propannitril, Hydronitrat | Fp.: 214-215°C (Z) |

(Z) = Zersetzung

- 15 -

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 40
Berliner Disconto-Bank AG, Berlin, Konto-Nr. 241/5008, Bankleitzahl 100 700
Berliner Handels-Gesellschaft – Frankfurter Bank –, Berlin,
Konto-Nr. 14-362, Bankleitzahl 100 202 00

54 FH IV 35

| Beispiel | Name der Verbindungen | Physikalische Konstante |
| --- | --- | --- |
| 15 | 2-(2-Chlorphenyl)-2-cyclohexyloxy-3-(1,2,4-triazol-1-yl)-propannitril | Fp.: 109-112°C |
| 16 | 2-(2-Chlorphenyl)-2-cyclohexyloxy-3-(1,2,4-triazol-1-yl)-propannitril, Hydronitrat | Fp.: 139°C (Z) |
| 17 | 2-Cyclopentyloxy-3-(imidazol-1-yl)-2-phenyl-propannitril | $n_D^{20}$: 1,5462 |
| 18 | 2-Cyclopentyloxy-3-(imidazol-1-yl)-2-phenyl-propannitril, Hydronitrat | Fp.: 164-167°C (Z) |
| 19 | 2-(2-Chlorphenyl)-2-cyclopentyloxy-3-(imidazol-1-yl)-propannitril | $n_D^{20}$: 1,5590 |
| 20 | 2-(2-Chlorphenyl)-2-cyclopentyloxy-3-(imidazol-1-yl)-propannitril, Hydronitrat | Fp.: 130°C (Z) |
| 21 | 2-Cyclopentyloxy-2-phenyl-3-(1,2,4-triazol-1-yl)-propannitril | Fp.: 85- 88°C |
| 22 | 2-Cyclopentyloxy-2-phenyl-3-(1,2,4-triazol-1-yl)-propannitril,Hydronitrat | Fp.: 172-174°C |
| 23 | 2-(2-Chlorphenyl)-2-cyclopentyloxy-3-(1,2,4-triazol-1-yl)-propannitril | Fp.: 91- 94°C |
| 24 | 2-(2-Chlorphenyl)-2-cyclopentyloxy-3-(1,2,4-triazol-1-yl)-propannitril, Hydronitrat | Fp.: 136°C (Z) |
| 25 | 2-Cyclohexyloxy-2-(2,4-dichlorphenyl)-3-(imidazol-1-yl)-propannitril | $n_D^{20}$: 1,5565 |
| 26 | 2-Cyclohexyloxy-2-(2,4-dichlorphenyl)-3-(imidazol-1-yl)-propannitril, Hydronitrat | Fp.: 156°C (Z) |
| 27 | 2-(4-Chlorphenyl)-2-cyclohexyloxy-3-(1,2,4-triazol-1-yl)-propannitril | Fp.: 97°C (Z) |

(Z) = Zersetzung

- 16 -

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 3571E

| Beispiel | Name der Verbindungen | Physikalische Konstante |
|---|---|---|
| 28 | 2-(4-Chlorphenyl)-2-cyclohexyloxy-3-(1,2,4-triazol-1-yl)-propannitril, Hydronitrat | Fp.: 176-178°C (Z) |
| 29 | 2-Cyclohexyloxy-2-(2,4-dichlorphenyl)-3-(1,2,4-triazol-1-yl)-propannitril | Fp.: 121-124°C |
| 30 | 2-Cyclohexyloxy-2-(2,4-dichlorphenyl)-3-(1,2,4-triazol-1-yl)-propannitril, Hydronitrat | Fp.: 145-148°C (Z) |
| 31 | 2-(4-Chlorphenyl)-2-cyclopentyloxy-3-(imidazol-1-yl)-propannitril | Fp.: 100-102°C |
| 32 | 2-(4-Chlorphenyl)-2-cyclopentyloxy-3-(imidazol-1-yl)-propan-nitril, Hydronitrat | Fp.: 200-201°C (Z) |
| 33 | 2-(4-Chlorphenyl)-2-cyclopentyloxy-3-(1,2,4-triazol-1-yl)-propan-nitril | Fp.: 89- 92°C |
| 34 | 2-(4-Chlorphenyl)-2-cyclopentyloxy-3-(1,2,4-triazol-1-yl)-propannitril, Hydronitrat | Fp.: 162-165°C (Z) |
| 35 | 2-Cyclopentyloxy-2-(2-fluorphenyl)-3-(imidazol-1-yl)-propannitril | $n_D^{20}$: 1,5328 |
| 36 | 2-Cyclopentyloxy-2-(2-fluorphenyl)-3-(1,2,4-triazol-1-yl)-propannitril | Fp.: 60- 62°C |
| 37 | 2-Cyclohexyloxy-2-(2-fluorphenyl)-3-(imidazol-1-yl)-pentannitril | $n_D^{20}$: 1,5300 |
| 38 | 2-Cyclohexyloxy-2-(2-fluorphenyl)-3-(1,2,4-triazol-1-yl)-propannitril | Fp.: 80- 82°C |
| 39 | 2-Cyclopentyloxy-2-(2-fluorphenyl)-3-(imidazol-1-yl)-propannitril, Hydronitrat | Fp.: 165- 68°C (Z) |
| 40 | 2-Cyclohexyloxy-2-(2-fluorphenyl)-3-(imidazol-1-yl)-propannitril, Hydronitrat | Fp.: 205-206°C (Z) |
| 41 | 2-Cyclopentyloxy-2-(2-methylphenyl)-3-(1,2,4-triazol-1-yl)-propannitril | Fp.: 78- 81°C |

(Z) = Zersetzung

- 17 -

Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 10
Berliner Disconto-Bank AG, Berlin, Konto-Nr. 241/5008, Bankleitzahl 100 7
Berliner Handels-Gesellschaft – Frankfurter Bank –, Berlin,
Konto-Nr. 14-362, Bankleitzahl 100 202 00

54 FH IV

Die erfindungsgemäßen Azolylpropannitrile sowie deren Säureadditionssalze sind fast farb- und geruchlose Öle oder Feststoffe. Die Salze lösen sich zum kleinen Teil in Wasser und gut in polaren organischen Lösungsmitteln wie Acetonitril, N,N-Dimethylformamid, niederen Alkoholen, Chloroform und Methylenchlorid. Die freien Basen lösen sich schlecht in Wasser und mehr oder weniger gut in organischen Lösungsmitteln wie zum Beispiel Alkoholen, Äthern oder chlorierten Kohlenwasserstoffen.

Die als Ausgangsmaterialien zu verwendenden 3-Aryl- beziehungsweise 3-Alkylsulfonyloxy-propannitrile der allgemeinen Formel II mit X in der Bedeutung Aryl- beziehungsweise Alkylsulfonyloxy sind in der Literatur bislang nicht beschrieben. Man erhält sie, indem man die bislang literaturunbekannten Phenylacetonitrile der allgemeinen Formel

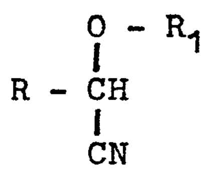

III

wobei R und $R_1$ die oben angegebenen Bedeutungen haben, nach an sich bekannten Methoden hydroxymethyliert und die so erhaltenen 3-Hydroxypropannitrile der allgemeinen Formel

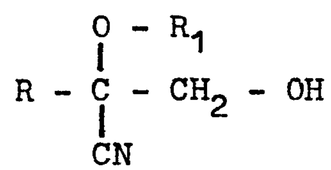

IV

mit geeigneten Sulfonsäurederivaten, wie zum Beispiel Sulfonsäurechlorid, gegebenenfalls unter Zusatz eines Säurebinders, umgesetzt. Die Hydroxymethylverbindungen der allgemeinen Formel IV sind bisher nicht literaturbekannt. Die Phenylacetonitrile der allgemeinen Formel III erhält man durch Umsetzung von



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

O-sulfonierten Cyanhydrinen mit den entsprechenden Alkoholen $R_1OH$.

Die folgenden Ausführungsbeispiele dienen zur Erläuterung der Anwendungsmöglichkeiten der erfindungsgemäßen Verbindungen, die in Form der oben angeführten Zubereitungen erfolgte.

In ppm (Teile pro million Teile Spritzflüssigkeit) ist die Anwendungskonzentration der Wirkstoffe angegeben. Aus dem jeweils gefundenen Befall wurde die Fungizidwirkung in den nachfolgenden Beispielen wie folgt berechnet:

$$100 - \frac{\text{Befall in Behandelt} \cdot 100}{\text{Befall in Unbehandelt}} = \%\ \text{Wirkung}$$



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzah
Berliner Disconto-Bank AG, Berlin, Konto-Nr. 241/5008, Bankleitzahl 1
Berliner Handels-Gesellschaft – Frankfurter Bank –, Berlin,
Konto-Nr. 14-362, Bankleitzahl 100 202 00

S4 FI

## Beispiel 42

Wirkung prophylaktischer Blattbehandlung gegen Echten Mehltau Erysiphe graminis an Gerste im Gewächshaus

Junge Gerstenpflanzen im Stadium des ersten Blattes wurden mit den angegebenen Konzentrationen tropfnaß gespritzt. Nach Antrocknen der Spritzbeläge wurden die behandelten Pflanzen sowie unbehandelte Kontrollpflanzen mit trockenen Mehltausporen inokuliert, indem man die Versuchspflänzchen mit befallenen Pflanzen überstrich. Danach wurden die Versuchspflanzen im Gewächshaus bei etwa 20 bis 22° C kultiviert und nach einer Woche auf Prozent Befall der Blattfläche bonitiert. Die ausgezeichneten Wirkungen sind aus den Versuchsdaten ersichtlich.



Formular-Nr.: 1439-2
Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5006, Bankleitzahl 100 700 00
Berliner Handels-Gesellschaft — Frankfurter Bank —, Berlin,
Konto-Nr. 14-362, Bankleitzahl 100 202 00
54 FH IV 35718

% Wirkung gegen Erysiphe graminis mit Wirkstoffkonzentration

| Erfindungsgemäße Verbindungen | 250 | 25 | 2,5 ppm |
|---|---|---|---|
| 2-Cyclopentyloxy-3-(imidazol-1-yl)-2-(2-methylphenyl)-propannitril, Hydronitrat | 100 | 100 | 100 |
| 2-Cyclopentyloxy-2-(2-methylphenyl)-3-(1,2,4-triazol-1-yl)-propannitril, Hydronitrat | 100 | 100 | 98 |
| 2-Cyclohexyloxy-3-(imidazol-1-yl)-2-(2-methylphenyl)-propannitril, Hydronitrat | 100 | 100 | 100 |
| 2-Cyclohexyloxy-2-(2-methylphenyl)-3-(1,2,4-triazol-1-yl)-propannitril, Hydronitrat | 100 | 100 | >90 |
| 2-Cyclohexyloxy-3-(imidazol-1-yl)-2-(2-methylphenyl)-propannitril | 100 | 100 | 100 |
| 2-Cyclohexyloxy-2-(2-methylphenyl)-3-(1,2,4-triazol-1-yl)-propannitril | 100 | 100 | >90 |
| 2-Cyclopentyloxy-3-(imidazol-1-yl)-2-(2-methylphenyl)-propannitril | 100 | 100 | 100 |
| 2-Cyclopentyloxy-2-(2-methylphenyl)-3-(1,2,4-triazol-1-yl)-propannitril | 100 | 100 | 100 |
| 2-Cyclohexyloxy-3-(imidazol-1-yl)-2-phenyl-propannitril | 100 | 100 | 100 |
| 2-Cyclohexyloxy-3-(imidazol-1-yl)-2-phenyl-propannitril, Hydronitrat | 100 | 100 | 100 |
| 2-(2-Chlorphenyl)-2-cyclohexyloxy-3-(imidazol-1-yl)-propannitril | 100 | 100 | 95 |
| 2-(2-Chlorphenyl)-2-cyclohexyloxy-3-(imidazol-1-yl)-propannitril, Hydronitrat | 100 | 100 | 100 |

- 21

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl
Berliner Handels-Gesellschaft – Frankfurter Bank –, Berlin,
Konto-Nr. 14-362, Bankleitzahl 100 202 00

54

% Wirkung gegen Erysiphe graminis mit Wirkstoffkonzentration

| Erfindungsgemäße Verbindungen | 250 | 25 | 2,5 ppm |
|---|---|---|---|
| 2-Cyclohexyloxy-2-phenyl-3-(1,2,4-triazol-1-yl)-propannitril | 100 | 100 | 87 |
| 2-Cyclohexyloxy-2-phenyl-3-(1,2,4-triazol-1-yl)-propannitril, Hydronitrat | 100 | 100 | 100 |
| 2-(4-Chlorphenyl)-2-cyclohexyloxy-3-(imidazol-1-yl)-propannitril, Hydronitrat | 100 | 100 | 100 |
| 2-(2-Chlorphenyl)-2-cyclohexyloxy-3-(1,2,4-triazol-1-yl)-propannitril | 100 | 100 | 100 |
| 2-(2-Chlorphenyl)-2-cyclohexyloxy-3-(1,2,4-triazol-1-yl)-propannitril, Hydronitrat | 100 | 100 | 100 |
| 2-Cyclopentyloxy-3-(imidazol-1-yl)-2-phenyl-propannitril | 100 | 100 | 95 |
| 2-Cyclopentyloxy-3-(imidazol-1-yl)-2-phenyl-propannitril, Hydronitrat | 100 | 100 | 100 |
| 2-(2-Chlorphenyl)-2-cyclopentyloxy-3-(imidazol-1-yl)-propannitril | 100 | 100 | 100 |
| 2-(2-Chlorphenyl)-2-cyclopentyloxy-3-(imidazol-1-yl)-propannitril, Hydronitrat | 100 | 100 | 100 |
| 2-Cyclopentyloxy-2-phenyl-3-(1,2,4-triazol-1-yl)-propannitril | 100 | 100 | 100 |
| 2-Cyclopentyloxy-2-phenyl-3-(1,2,4-triazol-1-yl)-propannitril,Hydronitrat | 100 | 100 | 100 |
| 2-(2-Chlorphenyl)-2-cyclopentyloxy-3-(1,2,4-triazol-1-yl)-propannitril | 100 | 100 | 87 |
| 2-(2-Chlorphenyl)-2-cyclopentyloxy-3-(1,2,4-triazol-1-yl)-propannitril, Hydronitrat | 100 | 100 | - |

- 22 -

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

| Erfindungsgemäße Verbindungen | % Wirkung gegen Erysiphe graminis mit Wirkstoffkonzentration 250 | 25 | 2,5 ppm |
|---|---|---|---|
| 2-Cyclohexyloxy-2-(2,4-dichlorphenyl)-3-(imidazol-1-yl)-propannitril | 100 | 100 | 87 |
| 2-Cyclohexyloxy-2-(2,4-dichlorphenyl)-3-(imidazol-1-yl)-propannitril, Hydronitrat | 100 | 100 | 88 |
| 2-(4-Chlorphenyl)-2-cyclohexyloxy-3-(1,2,4-triazol-1-yl)-propannitril | 100 | 100 | 100 |
| 2-(4-Chlorphenyl)-2-cyclohexyloxy-3-(1,2,4-triazol-1-yl)-propannitril, Hydronitrat | 100 | 100 | 94 |
| 2-Cyclohexyloxy-2-(2,4-dichlorphenyl)-3-(1,2,4-triazol-1-yl)-propannitril | 100 | 100 | 100 |
| 2-Cyclohexyloxy-2-(2,4-dichlorphenyl)-3-(1,2,4-triazol-1-yl)-propannitril, Hydronitrat | 98 | 94 | 92 |
| 2-(4-Chlorphenyl)-2-cyclopentyloxy-3-(imidazol-1-yl)-propannitril | 100 | 100 | 100 |
| 2-(4-Chlorphenyl)-2-cyclopentyloxy-3-(imidazol-1-yl)-propannitril, Hydronitrat | 100 | 100 | 100 |
| 2-(4-Chlorphenyl)-2-cyclopentyloxy-3-(1,2,4-triazol-1-yl)-propannitril | 100 | 100 | 100 |
| 2-(4-Chlorphenyl)-2-cyclopentyloxy-3-(1,2,4-triazol-1-yl)-propannitril, Hydronitrat | 100 | 100 | 100 |

Die erfindungsgemäßen Mittel lagen als 10 %ige oder als 20 %ige, in Wasser dispergierbare Formulierungen vor.

- 23 -

B e i s p i e l 43

Wirkung prophylaktischer Blattbehandlung gegen Erysiphe cichoracearum an Kürbispflanzen im Gewächshaus.

Mit den angegebenen Wirkstoffkonzentrationen tropfnaß gespritzte Kürbispflanzen wurden nach Antrocknen des Spritzbelages durch Aufstäuben trockener Mehltausporen von Erysiphe cichoracearum inokuliert und zusammen mit inokulierten unbehandelten Kontrollpflanzen im Gewächshaus bei 24°C inkubiert. Nach einer Woche wurde die befallene Mehltaufläche in % von der gesamten Blattfläche geschätzt.

Die Anmerkung "s" in der Tabelle bedeutet, die Fungizidwirkung ließ sich auch am Zuwachs der Pflanzen (nach der Behandlung) beurteilen und wurde positiv gefunden. Das heißt, die Wirkstoffe wurden systemisch in den Zuwachs transportiert und hatten auch an diesem hundertprozentige Wirkung gegen Mehltau.



Formular-Nr.: 1439-2
Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00
54 FH IV 35718

Gewerblicher Rechtsschutz

0134515

| Erfindungsgemäße Verbindungen | % Wirkung gegen Erysiphe cichoracearum mit Wirkstoffkonzentration 5 | 0,5 ppm |
|---|---|---|
| 2-Cyclopentyloxy-3-(imidazol-1-yl)-2-(2-methylphenyl)-propannitril, Hydronitrat | 100s | 100 |
| 2-Cyclopentyloxy-2-(2-methylphenyl)-3-(1,2,4-triazol-1-yl)-propannitril, Hydronitrat | 100s | 100s |
| 2-Cyclohexyloxy-3-(imidazol-1-yl)-2-(2-methylphenyl)-propannitril, Hydronitrat | 100s | 100 |
| 2-Cyclohexyloxy-2-(2-methylphenyl)-3-(1,2,4-triazol-1-yl)-propannitril, Hydronitrat | 100s | 100s |
| 2-Cyclohexyloxy-3-(imidazol-1-yl)-2-(2-methylphenyl)-propannitril | 100s | 100 |
| 2-Cyclohexyloxy-2-(2-methylphenyl)-3-(1,2,4-triazol-1-yl)-propannitril | 100s | 100s |
| 2-Cyclopentyloxy-3-(imidazol-1-yl)-2-(2-methylphenyl)-propannitril | 100s | 100s |
| 2-Cyclopentyloxy-2-(2-methylphenyl)-3-(1,2,4-triazol-1-yl)-propannitril | 100s | 100s |
| 2-Cyclohexyloxy-3-(imidazol-1-yl)-2-phenyl-propannitril | 100s | 100s |
| 2-Cyclohexyloxy-3-(imidazol-1-yl)-2-phenyl-propannitril, Hydronitrat | 100s | 100 |
| 2-(2-Chlorphenyl)-2-cyclohexyloxy-3-(imidazol-1-yl)-propannitril | 100 | 100 |
| 2-(2-Chlorphenyl)-2-cyclohexyloxy-3-(imidazol-1-yl)-propannitril, Hydronitrat | 100 | 100 |

- 25 -

Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 4
Berliner Disconto-Bank AG, Berlin, Konto-Nr. 241/5008, Bankleitzahl 100 70
Berliner Handels-Gesellschaft – Frankfurter Bank –, Berlin,
Konto-Nr. 14-362, Bankleitzahl 100 202 00

54 FH IV 3

% Wirkung gegen Erysiphe cichoracearum mit Wirkstoffkonzentration

| Erfindungsgemäße Verbindungen | 5 | 0,5 ppm |
|---|---|---|
| 2-Cyclohexyloxy-2-phenyl-3-(1,2,4-triazol-1-yl)-propannitril | 100 | 100 |
| 2-Cyclohexyloxy-2-phenyl-3-(1,2,4-triazol-1-yl)-propannitril, Hydronitrat | 100 | 100 |
| 2-(4-Chlorphenyl)-2-cyclohexyloxy-3-(imidazol-1-yl)-propan-nitril, Hydronitrat | 100 | 100 |
| 2-(2-Chlorphenyl)-2-cyclohexyloxy-3-(1,2,4-triazol-1-yl)-propan-nitril | 100 | 100 |
| 2-(2-Chlorphenyl)-2-cyclohexyloxy-3-(1,2,4-triazol-1-yl)-propan-nitril, Hydronitrat | 100 | 100 |
| 2-Cyclopentyloxy-3-(imidazol-1-yl)-2-phenyl-propannitril | 100 | 100 |
| 2-Cyclopentyloxy-3-(imidazol-1-yl)-2-phenyl-propannitril, Hydro-nitrat | 100 | 100 |
| 2-(2-Chlorphenyl)-2-cyclopentyloxy-3-(imidazol-1-yl)-propannitril | 100 | 100 |
| 2-(2-Chlorphenyl)-2-cyclopentyloxy-3-(imidazol-1-yl)-propan-nitril, Hydronitrat | 100 | 97 |
| 2-Cyclopentyloxy-2-phenyl-3-(1,2,4-triazol-1-yl)-propannitril | 100 | 100 |
| 2-Cyclopentyloxy-2-phenyl-3-(1,2,4-triazol-1-yl)-propan-nitril,Hydronitrat | 100 | 100 |
| 2-(2-Chlorphenyl)-2-cyclopentyloxy-3-(1,2,4-triazol-1-yl)-propan-nitril | 97 | 95 |
| 2-(2-Chlorphenyl)-2-cyclopentyloxy-3-(1,2,4-triazol-1-yl)-propan-nitril, Hydronitrat | 100 | 100 |

- 26 -

Gewerblicher Rechtsschutz

0134515

% Wirkung gegen Erysiphe cichoracearum
mit Wirkstoffkonzentration

| Erfindungsgemäße Verbindungen | 5 | 0,5 ppm |
|---|---|---|
| 2-Cyclohexyloxy-2-(2,4-dichlorphenyl)-3-(imidazol-1-yl)-propannitril | 100 | 100 |
| 2-Cyclohexyloxy-2-(2,4-dichlorphenyl)-3-(imidazol-1-yl)-propannitril, Hydronitrat | 100 | 98 |
| 2-(4-Chlorphenyl)-2-cyclohexyloxy-3-(1,2,4-triazol-1-yl)-propannitril | 100 | 100 |
| 2-(4-Chlorphenyl)-2-cyclohexyloxy-3-(1,2,4-triazol-1-yl)-propannitril, Hydronitrat | 100 | 100 |
| 2-Cyclohexyloxy-2-(2,4-dichlorphenyl)-3-(1,2,4-triazol-1-yl)-propannitril | 100 | 100 |
| 2-Cyclohexyloxy-2-(2,4-dichlorphenyl)-3-(1,2,4-triazol-1-yl)-propannitril, Hydronitrat | 100 | 96 |
| 2-(4-Chlorphenyl)-2-cyclopentyloxy-3-(imidazol-1-yl)-propannitril | 100 | 100 |
| 2-(4-Chlorphenyl)-2-cyclopentyloxy-3-(imidazol-1-yl)-propannitril, Hydronitrat | 100 | 100 |
| 2-(4-Chlorphenyl)-2-cyclopentyloxy-3-(1,2,4-triazol-1-yl)-propannitril | 100s | 100 |
| 2-(4-Chlorphenyl)-2-cyclopentyloxy-3-(1,2,4-triazol-1-yl)-propannitril, Hydronitrat | 100s | 100 |

| Vergleichsmittel | 20 | 4 ppm |
|---|---|---|
| 2-(1-Methyl-n-heptyl)-4,6-dinitrophenol-crotonat | 99,5 | 98,5 |

Die erfindungsgemäßen Mittel lagen als 20 %ige, in Wasser dispergierbare Formulierungen vor.

- 27

Formular-Nr.: 1499-3

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 10(
Berliner Disconto-Bank AG, Berlin, Konto-Nr. 241/5008, Bankleitzahl 100 7
Berliner Handels-Gesellschaft — Frankfurter Bank —, Berlin,
Konto-Nr. 14-362, Bankleitzahl 100 202 00

SC3 GC

Gewe.bli... Rechtsschrift

0134515

## Beispiel 44

Wirkung prophylaktischer Blattbehandlung gegen Zwergrost Puccinia hordei an Gerste in klimatisierter Pflanzenwuchskammer

Junge Gerstenpflanzen im Stadium des ersten Blattes wurden mit angegebener Konzentration tropfnaß gespritzt. Nach Antrocknen der Spritzbeläge wurden die behandelten Pflanzen sowie unbehandelte Kontrollpflanzen durch Überstreichen mit zwergrostbefallenen Pflanzen inokuliert und in eine Pflanzenwuchskammer gebracht. Bei $15^{0}$ C und in den beiden ersten Tagen unter nahezu feuchtgesättigter Luft wurden die Pflanzen 10 Tage kultiviert. Danach wurde der prozentuale Anteil rostbefallener Blattfläche notiert.



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 4
Berliner Disconto-Bank AG, Berlin, Konto-Nr. 241/5008, Bankleitzahl 100 70
Berliner Handels-Gesellschaft — Frankfurter Bank —, Berlin,
Konto-Nr. 14-362, Bankleitzahl 100 202 00

54 FH IV 3

% Wirkung gegen Puccinia hordei mit Wirkstoffkonzentration

| Erfindungsgemäße Verbindungen | 500 | 250 | 25 ppm |
|---|---|---|---|
| 2-Cyclopentyloxy-3-(imidazol-1-yl)-2-(2-methylphenyl)-propannitril, Hydronitrat | 100 | 100 | >90 |
| 2-Cyclopentyloxy-2-(2-methylphenyl)-3-(1,2,4-triazol-1-yl)-propannitril, Hydronitrat | 100 | 98 | - |
| 2-Cyclohexyloxy-3-(imidazol-1-yl)-2-(2-methylphenyl)-propannitril, Hydronitrat | 100 | >90 | - |
| 2-Cyclohexyloxy-2-(2-methylphenyl)-3-(1,2,4-triazol-1-yl)-propannitril, Hydronitrat | 100 | - | - |
| 2-Cyclohexyloxy-3-(imidazol-1-yl)-2-(2-methylphenyl)-propannitril | 100 | - | - |
| 2-Cyclohexyloxy-2-(2-methylphenyl)-3-(1,2,4-triazol-1-yl)-propannitril | 100 | - | - |
| 2-Cyclopentyloxy-3-(imidazol-1-yl)-2-(2-methylphenyl)-propannitril | 100 | 100 | - |
| 2-Cyclopentyloxy-2-(2-methylphenyl)-3-(1,2,4-triazol-1-yl)-propannitril | 100 | 100 | - |
| 2-Cyclohexyloxy-3-(imidazol-1-yl)-2-phenyl-propannitril | 100 | 100 | - |
| 2-Cyclohexyloxy-3-(imidazol-1-yl)-2-phenyl-propannitril, Hydronitrat | - | 95 | - |
| 2-(2-Chlorphenyl)-2-cyclohexyloxy-3-(imidazol-1-yl)-propannitril | 100 | 100 | - |
| 2-(2-Chlorphenyl)-2-cyclohexyloxy-3-(imidazol-1-yl)-propannitril, Hydronitrat | - | 98 | - |



Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 1
Berliner Disconto-Bank AG, Berlin, Konto-Nr. 241/5008, Bankleitzahl 100
Berliner Handels-Gesellschaft — Frankfurter Bank —, Berlin,
Konto-Nr. 14-362, Bankleitzahl 100 202 00

54 FH I

% Wirkung gegen Puccinia hordei mit Wirkstoffkonzentration

| Erfindungsgemäße Verbindungen | 500 | 250 | 25 ppm |
|---|---|---|---|
| 2-Cyclohexyloxy-2-phenyl-3-(1,2,4-triazol-1-yl)-propannitril | - | 98 | - |
| 2-Cyclohexyloxy-2-phenyl-3-(1,2,4-triazol-1-yl)-propannitril, Hydronitrat | 100 | 100 | - |
| 2-(4-Chlorphenyl)-2-cyclohexyloxy-3-(imidazol-1-yl)-propannitril, Hydronitrat | 100 | 100 | 90 |
| 2-(2-Chlorphenyl)-2-cyclohexyloxy-3-(1,2,4-triazol-1-yl)-propannitril, Hydronitrat | - | 95 | - |
| 2-Cyclopentyloxy-3-(imidazol-1-yl)-2-phenyl-propannitril | 100 | 100 | 90 |
| 2-Cyclopentyloxy-3-(imidazol-1-yl)-2-phenyl-propannitril, Hydronitrat | 100 | 100 | - |
| 2-(2-Chlorphenyl)-2-cyclopentyloxy-3-(imidazol-1-yl)-propannitril | - | 95 | 93 |
| 2-(2-Chlorphenyl)-2-cyclopentyloxy-3-(imidazol-1-yl)-propannitril, Hydronitrat | 100 | 100 | 85 |
| 2-Cyclopentyloxy-2-phenyl-3-(1,2,4-triazol-1-yl)-propannitril | 100 | 100 | 95 |
| 2-Cyclopentyloxy-2-phenyl-3-(1,2,4-triazol-1-yl)-propannitril,Hydronitrat | - | 95 | - |
| 2-(2-Chlorphenyl)-2-cyclopentyloxy-3-(1,2,4-triazol-1-yl)-propannitril | 100 | 100 | 85 |
| 2-Cyclohexyloxy-2-(2,4-dichlorphenyl)-3-(imidazol-1-yl)-propannitril | 100 | 100 | 85 |



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

% Wirkung gegen Puccinia hordei mit Wirkstoffkonzentration

| Erfindungsgemäße Verbindungen | 500 | 250 | 25 ppm |
|---|---|---|---|
| 2-Cyclohexyloxy-2-(2,4-dichlorphenyl)-3-(imidazol-1-yl)-propannitril, Hydronitrat | 100 | 100 | - |
| 2-(4-Chlorphenyl)-2-cyclohexyloxy-3-(1,2,4-triazol-1-yl)-propannitril | 100 | 100 | - |
| 2-(4-Chlorphenyl)-2-cyclohexyloxy-3-(1,2,4-triazol-1-yl)-propannitril, Hydronitrat | 100 | 100 | 95 |
| 2-Cyclohexyloxy-2-(2,4-dichlorphenyl)-3-(1,2,4-triazol-1-yl)-propannitril | 100 | 100 | - |
| 2-Cyclohexyloxy-2-(2,4-dichlorphenyl)-3-(1,2,4-triazol-1-yl)-propannitril, Hydronitrat | - | 95 | - |
| 2-(4-Chlorphenyl)-2-cyclopentyloxy-3-(imidazol-1-yl)-propannitril | 100 | 100 | 100 |
| 2-(4-Chlorphenyl)-2-cyclopentyloxy-3-(imidazol-1-yl)-propannitril, Hydronitrat | 100 | 100 | 100 |
| 2-(4-Chlorphenyl)-2-cyclopentyloxy-3-(1,2,4-triazol-1-yl)-propannitril | 100 | 100 | 100 |
| 2-(4-Chlorphenyl)-2-cyclopentyloxy-3-(1,2,4-triazol-1-yl)-propannitril, Hydronitrat | 100 | 100 | 100 |
| <u>Vergleichsmittel</u> | | | |
| 2,3-Dihydro-5-carboxanilido-6-methyl-1,4-oxathiin-4,4-dioxid | | 97,5 | <75 |

Die erfindungsgemäßen Mittel lagen als 10 %ige oder als 20 %ige, in Wasser dispergierbare Formulierungen vor.

- 31

Gewerblicher Rechtsschutz

0134515

B e i s p i e l 45

Wirkung prophylaktischer Blattbehandlung gegen die Blattfleckenkrankheit Cercospora beticola an Zuckerrüben (Beta vulgaris)

Zuckerrübenpflanzen mit 4 gut entwickelten Laubblättern wurden mit den angegebenen Konzentrationen tropfnaß gespritzt. Nach dem Antrocknen des Spritzbelages wurden die behandelten Pflanzen sowie unbehandelte Kontrollpflanzen mit einer Suspension von 15 000 Cercospora-Sporen pro Milliliter gleichmäßig besprüht. Bei 26° C und wasserdampfgesättigter Luft wurden die Pflanzen vier Tage im Gewächshaus inkubiert und danach bei etwa 22° C für weitere zehn Tage im Gewächshaus gehalten. Dann wurde der Anteil befallener Blattfläche notiert.



Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

% Wirkung gegen Cercospora beticola mit Wirkstoffkonzentration

| Erfindungsgemäße Verbindungen | 500 | 100 ppm |
|---|---|---|
| 2-Cyclohexyloxy-3-(imidazol-1-yl)-2-phenyl-propannitril | 100 | - |
| 2-Cyclohexyloxy-3-(imidazol-1-yl)-2-phenyl-propannitril, Hydronitrat | 99 | 93 |
| 2-(2-Chlorphenyl)-2-cyclohexyloxy-3-(imidazol-1-yl)-propannitril | 93 | - |
| 2-(2-Chlorphenyl)-2-cyclohexyloxy-3-(imidazol-1-yl)-propannitril, Hydronitrat | 97 | 86 |
| 2-Cyclohexyloxy-2-phenyl-3-(1,2,4-triazol-1-yl)-propannitril | 100 | 90 |
| 2-Cyclohexyloxy-2-phenyl-3-(1,2,4-triazol-1-yl)-propannitril, Hydronitrat | 100 | 100 |
| 2-(2-Chlorphenyl)-2-cyclohexyloxy-3-(1,2,4-triazol-1-yl)-propannitril | 100 | 83 |
| 2-(2-Chlorphenyl)-2-cyclohexyloxy-3-(1,2,4-triazol-1-yl)-propannitril, Hydronitrat | 100 | - |
| 2-Cyclopentyloxy-3-(imidazol-1-yl)-2-phenyl-propannitril | 100 | 99 |
| 2-Cyclopentyloxy-3-(imidazol-1-yl)-2-phenyl-propannitril, Hydronitrat | 100 | 96 |
| 2-(2-Chlorphenyl)-2-cyclopentyloxy-3-(imidazol-1-yl)-propannitril | 100 | 99 |
| 2-(2-Chlorphenyl)-2-cyclopentyloxy-3-(imidazol-1-yl)-propannitril, Hydronitrat | 100 | 99 |
| 2-Cyclopentyloxy-2-phenyl-3-(1,2,4-triazol-1-yl)-propannitril | 100 | 100 |



Formular-Nr.: 1438-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 1(
Berliner Disconto-Bank AG, Berlin, Konto-Nr. 241/5008, Bankleitzahl 1(
Berliner Handels-Gesellschaft — Frankfurter Bank —, Berlin,
Konto-Nr. 14-362, Bankleitzahl 100 202 00

54 FH

| Erfindungsgemäße Verbindungen | % Wirkung gegen Cercospora beticola mit Wirkstoffkonzentration 500 | 100 ppm |
|---|---|---|
| 2-Cyclopentyloxy-2-phenyl-3-(1,2,4-triazol-1-yl)-propannitril,Hydronitrat | 100 | 100 |
| 2-(2-Chlorphenyl)-2-cyclopentyloxy-3-(1,2,4-triazol-1-yl)-propannitril | 100 | 100 |
| 2-(2-Chlorphenyl)-2-cyclopentyloxy-3-(1,2,4-triazol-1-yl)-propannitril, Hydronitrat | 100 | 100 |
| 2-Cyclohexyloxy-2-(2,4-dichlorphenyl)-3-(imidazol-1-yl)-propannitril | 99 | 93 |
| 2-Cyclohexyloxy-2-(2,4-dichlorphenyl)-3-(imidazol-1-yl)-propannitril, Hydronitrat | 93 | 80 |
| 2-(4-Chlorphenyl)-2-cyclohexyloxy-3-(1,2,4-triazol-1-yl)-propannitril | 100 | 84 |
| 2-(4-Chlorphenyl)-2-cyclohexyloxy-3-(1,2,4-triazol-1-yl)-propannitril, Hydronitrat | 100 | 82 |
| 2-Cyclohexyloxy-2-(2,4-dichlorphenyl)-3-(1,2,4-triazol-1-yl)-propannitril | 100 | - |
| 2-(4-Chlorphenyl)-2-cyclopentyloxy-3-(imidazol-1-yl)-propannitril | 100 | 96 |
| 2-(4-Chlorphenyl)-2-cyclopentyloxy-3-(imidazol-1-yl)-propannitril, Hydronitrat | 100 | 100 |
| 2-(4-Chlorphenyl)-2-cyclopentyloxy-3-(1,2,4-triazol-1-yl)-propannitril | 100 | 100 |
| 2-(4-Chlorphenyl)-2-cyclopentyloxy-3-(1,2,4-triazol-1-yl)-propannitril, Hydronitrat | 100 | 96 |

Die erfindungsgemäßen Mittel lagen als 10 %ige oder als 20 %ige, in Wasser dispergierbare Formulierungen vor.



Formular-Nr.: 1439-2
Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin, Konto-Nr. 14-362, Bankleitzahl 100 202 00
54 FH IV 35718

0134515

## Beispiel 46

Wirkung prophylaktischer Spritzbehandlung gegen Fusarium culmorum an Kolbenhirse (Setaria italica)

Junge Hirsepflanzen von 3 bis 4 cm Höhe wurden mit angegebener Konzentration tropfnaß gespritzt. Nach Antrocknen des Spritzbelages wurden die behandelten Pflanzen sowie unbehandelte Kontrollpflanzen mit einer 30 % Biomalz enthaltenden wäßrigen Suspension der Fusariumsporen (800 000 pro Milliliter) besprüht und feucht im Gewächshaus bei 20 bis 22° C inkubiert. Nach 6 Tagen wurde der prozentuale Anteil befallener Blattfläche notiert.

- 35

Formular-Nr.: 1459-2
Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitz
Berliner Disconto-Bank AG, Berlin, Konto-Nr. 241/5008, Bankleitzah
Berliner Handels-Gesellschaft – Frankfurter Bank –, Berlin,
Konto-Nr. 14-362, Bankleitzahl 100 202 00
S4

SCHERING AG
Gewerblicher Rechtsschutz



% Wirkung gegen Fusarium culmorum mit Wirkstoffkonzentration

| Erfindungsgemäße Verbindungen | 500 ppm |
|---|---|
| 2-Cyclopentyloxy-3-(imidazol-1-yl)-2-(2-methylphenyl)-propannitril, Hydronitrat | 97 |
| 2-Cyclopentyloxy-2-(2-methylphenyl)-3-(1,2,4-triazol-1-yl)-propannitril, Hydronitrat | 97 |
| 2-Cyclohexyloxy-3-(imidazol-1-yl)-2-(2-methylphenyl)-propannitril, Hydronitrat | 96 |
| 2-Cyclohexyloxy-2-(2-methylphenyl)-3-(1,2,4-triazol-1-yl)-propannitril, Hydronitrat | 91 |
| 2-Cyclohexyloxy-3-(imidazol-1-yl)-2-(2-methylphenyl)-propannitril | 97 |
| 2-Cyclohexyloxy-2-(2-methylphenyl)-3-(1,2,4-triazol-1-yl)-propannitril | 96 |
| 2-Cyclopentyloxy-3-(imidazol-1-yl)-2-(2-methylphenyl)-propannitril | 99 |
| 2-Cyclopentyl oxy-2-(methylphenyl)-3-(1,2,4-triazol-1-yl)-propannitril | 97 |
| 2-Cyclopentyloxy-3-(imidazol-1-yl)-2-phenyl-propannitril | 95 |
| 2-Cyclopentyloxy-3-(imidazol-1-yl)-2-phenyl-propannitril, Hydronitrat | 90 |
| 2-(2-Chlorphenyl)-2-cyclopentyloxy-3-(imidazol-1-yl)-propannitril | 90 |
| 2-(2-Chlorphenyl)-2-cyclopentyloxy-3-(imidazol-1-yl)-propannitril, Hydronitrat | 93 |



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

% Wirkung gegen Fusarium culmorum mit Wirkstoffkonzentration

| Erfindungsgemäße Verbindungen | 500 ppm |
|---|---|
| 2-(4-Chlorphenyl)-2-cyclopentyloxy-3-(imidazol-1-yl)-propannitril | 97 |
| 2-(4-Chlorphenyl)-2-cyclopentyloxy-3-(imidazol-1-yl)-propannitril, Hydronitrat | 95 |
| 2-(4-Chlorphenyl)-2-cyclopentyloxy-3-(1,2,4-triazol-1-yl)-propannitril | 90 |
| 2-(4-Chlorphenyl)-2-cyclopentyloxy-3-(1,2,4-triazol-1-yl)-propannitril, Hydronitrat | 95 |

Die erfindungsgemäßen Mittel lagen als 10 %ige oder als 20 %ige, in Wasser dispergierbare Formulierungen vor.

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postf.
Postscheck-Konto: Berlin-West 1175-101, Bankleitzah
Berliner Commerzbank AG, Berlin, Konto-Nr. 108 700
Berliner Disconto-Bank AG, Berlin, Konto-Nr. 241/500
Berliner Handels-Gesellschaft — Frankfurter Bank —
Konto-Nr. 14-362, Bankleitzahl 100 202 00

## Beispiel 47

Wirkung prophylaktischer Spritzbehandlung gegen Botrytis cinerea an Tomatenpflanzen

Junge Tomatenpflanzen mit mindestens zwei gut entwickelten Laubblättern wurden mit der angegebenen Wirkstoffkonzentration tropfnaß gespritzt. Nach Antrocknen des Spritzbelages wurden die behandelten Pflanzen sowie unbehandelte Kontrollpflanzen inokuliert durch Aufsprühen einer Suspension von etwa 1 Million Botrytis-Sporen pro Milliliter Fruchsaftlösung. Anschließend wurden die Pflanzen feucht bei etwa 20° C im Gewächshaus inkubiert. Nach dem Zusammenbrechen der unbehandelten Pflanzen (= 100 % Befall) wurde der Befallsgrad der behandelten Pflanzen festgestellt.

| Erfindungsgemäße Verbindungen | % Wirkung gegen Botrytis cinerea mit Wirkstoffkonzentration 250 ppm |
|---|---|
| 2-(4-Chlorphenyl)-2-cyclopentyloxy-3-(imidazol-1-yl)-propannitril | 80 |
| 2-(4-Chlorphenyl)-2-cyclopentyloxy-3-(imidazol-1-yl)-propannitril, Hydronitrat | 80 |
| **Vergleichsmittel gemäß DE-OS 26 04 047** | |
| 2-(Imidazol-1-ylmethyl)-2-phenyl-hexannitril, Hydronitrat | 5 |

- 38 -

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin, Konto-Nr. 14-362, Bankleitzahl 100 202 00

54 FH IV 35718

## Beispiel 48

Wirkung prophylaktischer Spritzbehandlung gegen Helminthosporium teres (Pyrenophora teres) an Gerste

Junge Gerstenpflanzen im Stadium des ersten Blattes wurden mit der angegebenen Konzentration tropfnaß gespritzt. Nach Antrocknen des Spritzbelages wurden die behandelten Pflanzen sowie unbehandelte Kontrollpflanzen mit einer wäßrigen Konidiosporensuspension (etwa 50 000 pro Milliliter) von Helminthosporium teres besprüht und in einer Feuchtkammer im Gewächshaus 2 Tage bei 20 bis 22° C inkubiert. Danach wurden die Pflanzen im Gewächshaus bei 20 bis 22° C kultiviert. Eine Woche nach der Inokulation wurde der prozentuale Anteil befallener Blattfläche notiert.

% Wirkung gegen Helminthosporium teres mit Wirkstoffkonzentration

| Erfindungsgemäße Verbindungen | 500 ppm |
|---|---|
| 2-Cyclopentyloxy-3-(imidazol-1-yl)-2-(2-methylphenyl)-propannitril, Hydronitrat | 80 |
| 2-Cyclohexyloxy-3-(imidazol-1-yl)-2-(2-methylphenyl)-propannitril | 80 |
| 2-Cyclohexyloxy-2-(2-methylphenyl)-3-(1,2,4-triazol-1-yl)-propannitril | 80 |



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400
Berliner Disconto-Bank AG, Berlin, Konto-Nr. 241/5008, Bankleitzahl 100 700 0
Berliner Handels-Gesellschaft – Frankfurter Bank –, Berlin,
Konto-Nr. 14-362, Bankleitzahl 100 202 00

54 FH IV 3571

## B e i s p i e l 49

Wirkung prophylaktischer Spritzbehandlung gegen Cochliobolus miyabeanus an Reispflanzen

Junge Reispflanzen im Stadium des beginnenden zweiten Blattes wurden mit der angegebenen Konzentration tropfnaß gespritzt. Nach Antrocknen des Spritzbelages wurden die behandelten Pflanzen sowie unbehandelte Kontrollpflanzen mit einer wäßrigen Konidiensuspension (etwa 250 000 pro Milliliter) des Pilzes besprüht und im Gewächshaus bei 24° C feucht inkubiert. Nach 4 Tagen notierte man den prozentualen Anteil befallener Blattfläche.



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

S4 FH IV 35718

% Wirkung gegen Cochliobolus miyabeanus mit Wirkstoffkonzentration

| Erfindungsgemäße Verbindungen | 500 ppm |
|---|---|
| 2-Cyclopentyloxy-3-(imidazol-1-yl)-2-(2-methylphenyl)-propannitril, Hydronitrat | 80 |
| 2-Cyclopentyloxy-2-(2-methylphenyl)-3-(1,2,4-triazol-1-yl)-propannitril, Hydronitrat | 80 |
| 2-Cyclohexyloxy-3-(imidazol-1-yl)-2-(2-methylphenyl)-propannitril, Hydronitrat | 80 |
| 2-Cyclohexyloxy-2-(2-methylphenyl)-3-(1,2,4-triazol-1-yl)-propannitril, Hydronitrat | 80 |
| 2-Cyclohexyloxy-3-(imidazol-1-yl)-2-(2-methylphenyl)-propannitril | 80 |
| 2-Cyclohexyloxy-2-(2-methylphenyl)-3-(1,2,4-triazol-1-yl)-propannitril | 80 |
| 2-Cyclopentyloxy-3-(imidazol-1-yl)-2-(2-methylphenyl)-propannitril | 80 |



Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl 1
Berliner Handels-Gesellschaft — Frankfurter Bank —, Berlin,
Konto-Nr. 14-362, Bankleitzahl 100 202 00

54 Fl

## Beispiel 50

Wirkung prophylaktischer Spritzbehandlung gegen Phytophthora infestans an Tomatenpflanzen

Junge Tomatenpflanzen mit mindestens zwei gut entwickelten Laubblättern wurden tropfnaß gespritzt. Nach Antrocknen der Spritzbeläge wurden die behandelten Pflanzen sowie unbehandelte Kontrollpflanzen mit einer wäßrigen, 2 Stunden bei 11° C inkubierten Suspension von etwa 80 000 Phytophthora-Sporangien pro Milliliter besprüht. Bei etwa 18° C wurden die Pflanzen im Gewächshaus feucht inkubiert. Nach 5 Tagen wurde der prozentrale Anteil befallener Blattfläche notiert.

| Erfindungsgemäße Verbindung | % Wirkung gegen Phytophthora infestans mit 500 ppm Wirkstoffkonzentration |
|---|---|
| 2-Cyclohexyloxy-2-phenyl-3-(1,2,4-triazol-1-yl)-propannitril | 91 |

- 42 -

B e i s p i e l 51

Wirkung gegen Apfelschorf (Venturia inaequalis), prophylaktisch und kurativ

An Apfeltrieben im Freiland wurden die 5 jüngsten Blätter tropfnaß behandelt. Nach dem Antrocknen der Spritzbeläge wurden diese Triebe sowie nicht behandelte Triebe mit einer Suspension von 320 000 Konidiosporen des Pilzes pro Milliliter 3 %iger Traubenzuckerlösung in destilliertem Wasser besprüht und 53 Stunden lang in Polyäthylenbeutel unter Schattierung eingeschlossen. 8 Tage nach der Inokulation wurde eine Anzahl der unbehandelten, inokulierten Triebe einer kurativen Behandlung (tropfnaß) unterzogen. Fünf Wochen nach der Inokulation wurde der prozentuale Grad der Schorfbedeckung der Blattflächen notiert.

% Wirkung gegen Apfelschorf mit Wirkstoffkonzentration 750 ppm

| Erfindungsgemäße Verbindung | prophylaktisch | kurativ |
|---|---|---|
| 2-Cyclopentyloxy-3-(imidazol-1-yl)-2-(2-methylphenyl)-propannitril | 100 | 86 |
| Kontrolle (unbehandelt) | 35 % Befall | |



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl
Berliner Handels-Gesellschaft – Frankfurter Bank –, Berlin,
Konto-Nr. 14-362, Bankleitzahl 100 202 00

54 F

% Wirkung gegen Apfelschorf mit Wirkstoffkonzentration 300 ppm

| Erfindungsgemäße Verbindungen | prophylaktisch | kurativ |
|---|---|---|
| 2-Cyclopentyloxy-3-(imidazol-1-yl)-2-(2-methylphenyl)-propannitril | 86 | - |
| 2-Cyclohexyloxy-3-(imidazol-1-yl)-2-phenyl-propannitril | 98 | - |
| 2-Cyclohexyloxy-3-(imidazol-1-yl)-2-phenyl-propannitril, Hydronitrat | 90 | - |
| 2-Cyclopentyloxy-3-(imidazol-1-yl)-2-phenyl-propannitril | 97 | 81 |
| 2-Cyclopentyloxy-3-(imidazol-1-yl)-2-phenyl-propannitril, Hydronitrat | 91 | 90 |
| 2-Cyclopentyloxy-2-phenyl-3-(1,2,4-triazol-1-yl)-propannitril | 100 | 95 |
| 2-Cyclopentyloxy-2-phenyl-3-(1,2,4-triazol-1-yl)-propannitril,Hydronitrat | 100 | 90 |
| 2-(2-Chlorphenyl)-2-cyclopentyloxy-3-(1,2,4-triazol-1-yl)-propannitril | 98 | 73 |
| 2-(2-Chlorphenyl)-2-cyclopentyloxy-3-(1,2,4-triazol-1-yl)-propannitril, Hydronitrat | 88 | - |
| 2-Cyclohexyloxy-2-(2,4-dichlorphenyl)-3-(imidazol-1-yl)-propannitril | 84 | - |
| 2-(4-Chlorphenyl)-2-cyclohexyloxy-3-(1,2,4-triazol-1-yl)-propannitril | 100 | 93 |
| 2-(4-Chlorphenyl)-2-cyclohexyloxy-3-(1,2,4-triazol-1-yl)-propannitril, Hydronitrat | 100 | 99,5 |



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

54 FH IV 35718

Gewerblicher Rechtsschutz

0134515

% Wirkung gegen Apfelschorf mit Wirkstoffkonzentration 300 ppm

| Erfindungsgemäße Verbindungen | prophylaktisch | kurativ |
|---|---|---|
| 2-(4-Chlorphenyl)-2-cyclopentyloxy-3-(imidazol-1-yl)-propannitril | 96 | 99 |
| 2-(4-Chlorphenyl)-2-cyclopentyloxy-3-(imidazol-1-yl)-propannitril, Hydronitrat | 89 | 95 |
| 2-(4-Chlorphenyl)-2-cyclopentyloxy-3-(1,2,4-triazol-1-yl)-propannitril | 99,5 | 94 |
| 2-(4-Chlorphenyl)-2-cyclopentyloxy-3-(1,2,4-triazol-1-yl)-propannitril, Hydronitrat | 100 | 99 |

- 45

Formular-Nr.: 1489-8

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7000/00, Bankleitzahl 1(
Berliner Disconto-Bank AG, Berlin, Konto-Nr. 241/508, Bankleitzahl 100
Berliner Handels-Gesellschaft — Frankfurter Bank -, Berlin,
Konto-Nr. 14-362, Bankleitzahl 100 202 00

SC3 GC

Die folgenden Ausführungsbeispiele dienen zur Erläuterung der Anwendungsmöglichkeiten der erfindungsgemäßen Verbindungen als Wuchsregulatoren:

B e i s p i e l 52

Wachstumsregulatorische Effekte bei Zuckerrüben

Zuckerrüben wurden im Vorauflaufverfahren mit umgerechnet 0,5 kg Wirkstoff je ha behandelt und im Gewächshaus weiter kultiviert. 8 Tage nach der Applikation wurde die prozentuale Wuchshemmung als Präparateeinfluß festgehalten.

| Erfindungsgemäße Verbindungen | Prozentuale Wuchshemmung |
|---|---|
| 2-Cyclohexyloxy-2-(2-methylphenyl)-3-(1,2,4-triazol-1-yl)-propannitril, Hydronitrat | 40 |
| 2-Cyclohexyloxy-2-(2-methylphenyl)-3-(1,2,4-triazol-1-yl)-propannitril | 20 |
| Kontrolle | 0 |

Die Behandlung führte bei den Pflanzen zu einer Wuchshemmung und zu einer dunkelgrünen Färbung der Blätter.



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

## Beispiel 53

Wuchsregulation bei Zuckerrüben und Mais

Mais und Zuckerrüben wurden nach einer Vorauflauf-Spritzung mit den erfindungsgemäßen Substanzen im Gewächshaus angezogen. 11 Tage nach der Behandlung wurden wachstumsregulatorische Effekte anhand der prozentualen Wuchshemmung festgehalten. 0,5 kg Wirkstoff je ha führten zu den folgenden Ergebnissen:

| Erfindungsgemäße Verbindungen | Prozentuale Wuchshemmung Mais | Zuckerrübe |
|---|---|---|
| 2-(2-Chlorphenyl)-2-cyclohexyloxy-3-(imidazol-1-yl)-propannitril | 14 | 14 |
| 2-(2-Chlorphenyl)-2-cyclohexyloxy-3-(imidazol-1-yl)-propannitril, Hydronitrat | 21 | 14 |
| 2-(4-Chlorphenyl)-2-cyclohexyloxy-3-(imidazol-1-yl)-propannitril, Hydronitrat | 14 | 14 |
| 2-(4-Chlorphenyl)-2-cyclopentyloxy-3-(imidazol-1-yl)-propannitril | 21 | 29 |
| 2-(4-Chlorphenyl)-2-cyclopentyloxy-3-(1,2,4-triazol-1-yl)-propannitril | 36 | 57 |
| Kontrolle | 0 | 0 |

Es zeigt sich, daß die erfindungsgemäßen Substanzen deutlich das Längenwachstum der behandelten Pflanzen hemmen.



Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 1
Berliner Disconto-Bank AG, Berlin, Konto-Nr. 241/5008, Bankleitzahl 10
Berliner Handels-Gesellschaft – Frankfurter Bank –, Berlin,
Konto-Nr. 14-362, Bankleitzahl 100 202 00

54 FH

Beispiel 54

Wirkung prophylaktischer Blattbehandlung gegen Plasmopara viticola an Weinrebenpflanzen im Gewächshaus.

Junge Weinrebenpflanzen mit etwa 5 bis 8 Blättern wurden mit 500 ppm Wirkstoffkonzentration tropfnaß gespritzt, nach Antrocknen des Spritzbelages mit einer wäßrigen Aufschwemmung von Sporangien des Pilzes (etwa 55.000 pro ml) blattunterseits besprüht und sofort im Gewächshaus bei 22 bis 24° C und möglichst wasserdampfgesättigter Atmosphäre inkubiert.

Vom zweiten Tage an wurde die Luftfeuchtigkeit für 3 bis 4 Tage auf Normalhöhe zurückgenommen (30 bis 70 % Sättigung) und dann für einen Tag auf Wasserdampfsättigung gehalten. Anschließend wurde von jedem Blatt der prozentuale Anteil pilzbefallener Fläche notiert und der Durchschnitt je Behandlung zur Ermittlung der Fungizidwirkung wie oben beschrieben verrechnet.

| Erfindungsgemäße Verbindungen | % Wirkung gegen Plasmopara viticola |
|---|---|
| 2-Cyclopentyloxy-3-(imidazol-1-yl)-2-(2-methylphenyl)-propannitril, Hydronitrat | 89 |
| 2-Cyclopentyloxy-2-(2-methylphenyl)-3-(1,2,4-triazol-1-yl)-propannitril, Hydronitrat | 84 |
| 2-Cyclohexyloxy-3-(imidazol-1-yl)-2-(2-methylphenyl)-propannitril, Hydronitrat | 100 |
| 2-Cyclohexyloxy-2-(2-methylphenyl)-3-(1,2,4-triazol-1-yl)-propannitril, Hydronitrat | 85 |

- 48 -

| Erfindungsgemäße Verbindungen | % Wirkung gegen Plasmopara viticola |
|---|---|
| 2-Cyclohexyloxy-3-(imidazol-1-yl)-2-(2-methylphenyl)-propannitril | 95 |
| 2-Cyclohexyloxy-2-(2-methylphenyl)-3-(1,2,4-triazol-1-yl)-propannitril | 91 |
| 2-Cyclopentyloxy-3-(imidazol-1-yl)-2-(2-methylphenyl)-propannitril | 98 |
| 2-Cyclohexyloxy-2-phenyl-3-(1,2,4-triazol-1-yl)-propannitril | 91 |
| 2-Cyclopentyloxy-3-(imidazol-1-yl)-2-phenyl-propannitril | 100 |
| 2-Cyclopentyloxy-3-(imidazol-1-yl)-2-phenyl-propannitril, Hydronitrat | 73 |
| 2-(2-Chlorphenyl)-2-cyclopentyloxy-3-(imidazol-1-yl)-propannitril | 90 |
| 2-(2-Chlorphenyl)-2-cyclopentyloxy-3-(imidazol-1-yl)-propannitril, Hydronitrat | 97 |
| 2-Cyclopentyloxy-2-phenyl-3-(1,2,4-triazol-1-yl)-propannitril | 95 |
| 2-Cyclopentyloxy-2-phenyl-3-(1,2,4-triazol-1-yl)-propannitril,Hydronitrat | 98 |
| 2-(2-Chlorphenyl)-2-cyclopentyloxy-3-(1,2,4-triazol-1-yl)-propannitril | 98 |
| 2-(4-Chlorphenyl)-2-cyclopentyloxy-3-(imidazol-1-yl)-propannitril | 99,5 |
| 2-(4-Chlorphenyl)-2-cyclopentyloxy-3-(imidazol-1-yl)-propannitril, Hydronitrat | 100 |
| 2-(4-Chlorphenyl)-2-cyclopentyloxy-3-(1,2,4-triazol-1-yl)-propannitril | 97 |
| 2-(4-Chlorphenyl)-2-cyclopentyloxy-3-(1,2,4-triazol-1-yl)-propannitril, Hydronitrat | 85 |

Die erfindungsgemäßen Mittel lagen als 10 %ige oder als 20 %ige in Wasser dispergierbare Formulierungen vor.

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061
Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 1
Berliner Disconto-Bank AG, Berlin, Konto-Nr. 241/5008, Bankleitzahl 10
Berliner Handels-Gesellschaft – Frankfurter Bank –, Berlin,
Konto-Nr. 14-362, Bankleitzahl 100 202 00
Formular-Nr.: 1489-3
SC3 G

## Beispiel 55

Wirkung in vitro gegen Mucor spec. von Sojabohne

Wäßrige Wirkstoffzubereitungen wurden mit sterilisiertem, flüssigem Agarnährboden (2 % Biomalz, 2 % Agar) bei etwa 45° C in der Weise vermischt, daß die Mischung 250 ppm Aktivsubstanz enthielt. Die Mischung wurde in Polystyrol-Petrischalen von 8,5 cm Durchmesser 5 mm hoch eingegossen. Nach dem Erkalten des Nährbodens wurde in jede Petrischale ein mit Mucor bewachsenes Agarstück von 5 mm Durchmesser gesetzt. Je Behandlung wurden 2 Schalen und von Unbehandelt 4 Schalen beimpft.
Nach 5 Tagen Inkubation bei 22° C im Dunkel wurde der Koloniedurchmesser bestimmt und nach Abzug der Inokulatgröße von 5 mm wie folgt zur Berechnung der fungiziden Wirkung herangezogen:

$$100 - \frac{\text{Durchmesser ohne Inokulat in Behandelt} \cdot 100}{\text{Durchmesser ohne Inokulat in Unbehandelt}} = \text{\% Wirkung}$$

| Erfindungsgemäße Verbindungen | % Wirkung gegen Mucor spec. |
|---|---|
| 2-Cyclopentyloxy-2-(2-methylphenyl)-3-(1,2,4-triazol-1-yl)-propannitril, Hydronitrat | 80 |
| 2-Cyclopentyloxy-3-(imidazol-1-yl)-2-(2-methylphenyl)-propannitril | 75 |
| 2-Cyclohexyloxy-2-phenyl-3-(1,2,4-triazol-1-yl)-propannitril | 100 |
| 2-Cyclopentyloxy-3-(imidazol-1-yl)-2-phenyl-propannitril | 95 |
| 2-(2-Chlorphenyl)-2-cyclopentyloxy-3-(imidazol-1-yl)-propannitril | 94 |



Formular-Nr.: 1489-3

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft — Frankfurter Bank —, Berlin,
Konto-Nr. 14-362, Bankleitzahl 100 202 00

SC3 GC IV 42550

| Erfindungsgemäße Verbindungen | % Wirkung gegen Mucor spec. |
|---|---|
| 2-Cyclopentyloxy-2-phenyl-3-(1,2,4-triazol-1-yl)-propannitril | 100 |
| 2-Cyclopentyloxy-2-phenyl-3-(1,2,4-triazol-1-yl)-propannitril,Hydronitrat | 75 |
| 2-(2-Chlorphenyl)-2-cyclopentyloxy-3-(1,2,4-triazol-1-yl)-propannitril | 98 |
| 2-(4-Chlorphenyl)-2-cyclopentyloxy-3-(imidazol-1-yl)-propannitril | 100 |
| 2-(4-Chlorphenyl)-2-cyclopentyloxy-3-(imidazol-1-yl)-propannitril, Hydronitrat | 77 |
| 2-(4-Chlorphenyl)-2-cyclopentyloxy-3-(1,2,4-triazol-1-yl)-propannitril | 100 |
| 2-(4-Chlorphenyl)-2-cyclopentyloxy-3-(1,2,4-triazol-1-yl)-propannitril, Hydronitrat | 87 |
| Vergleichsmittel | |
| 2-Cyan-2-methoxyiminoessigsäure-äthylaminocarbonylamid | 50 |

Die erfindungsgemäßen Mittel lagen als 10 %ige oder als 20 %ige in Wasser dispergierbare Formulierungen vor.

- 51

Formular-Nr.: 1489-8

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl
Berliner Handels-Gesellschaft – Frankfurter Bank –, Berlin,
Konto-Nr. 14-362, Bankleitzahl 100 202 00

SC3

B e i s p i e l   56

Wirkung in vitro gegen Pseudomonas phaseolicola (Fettfleckenkrankheit der Bohne)

Wäßrige Wirkstoffzubereitungen wurden mit sterilisiertem, flüssigem Agarnährboden (2 % Biomalz, 2 % Agar) bei etwa $45^{o}$ C in der Weise vermischt, daß die Mischung 50 ppm Aktivsubstanz enthielt. Die Mischung wurde in Polystyrol-Petrischalen von 8,5 cm Durchmesser 5 mm hoch gegossen. Nach dem Erkalten des Nährbodens wurde in die Mitte der Petrischale eine Suspension von Pseudomonas phaseolicola in destilliertem Wasser mittels 5 mm großer Impf-Öse abgetupft. Je Behandlung wurden 2 Schalen und von Unbehandelt 4 Schalen beimpft. Nach 4 1/2 Wochen Inkubation bei 20 - $22^{o}$ C im Dunkel wurde der Koloniedurchmesser bestimmt und nach Abzug der Inokulatgröße wie folgt zur Berechnung der bakteriziden Wirkung herangezogen:

$$100 - \frac{\text{Durchmesser ohne Inokulat in Behandelt} \cdot 100}{\text{Durchmesser ohne Inokulat in Unbehandelt}} = \text{\% Wirkung}$$

| Erfindungsgemäße Verbindungen | % Wirkung gegen Pseudomonas phaseolicola |
|---|---|
| 2-(2-Chlorphenyl)-2-cyclopentyloxy-3-(imidazol-1-yl)-propannitril, Hydronitrat | 82 |
| 2-Cyclopentyloxy-2-phenyl-3-(1,2,4-triazol-1-yl)-propannitril | 73 |
| 2-Cyclopentyloxy-2-phenyl-3-(1,2,4-triazol-1-yl)-propannitril,Hydronitrat | 91 |
| 2-(2-Chlorphenyl)-2-cyclopentyloxy-3-(1,2,4-triazol-1-yl)-propannitril | 91 |



Formular-Nr.: 1489-8

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

SC3 GC IV 42550

| Erfindungsgemäße Verbindungen | % Wirkung gegen Pseudomonas phaseolicola |
|---|---|
| 2-(4-Chlorphenyl)-2-cyclopentyloxy-3-(imidazol-1-yl)-propannitril | 100 |
| 2-(4-Chlorphenyl)-2-cyclopentyloxy-3-(imidazol-1-yl)-propannitril, Hydronitrat | 100 |
| 2-(4-Chlorphenyl)-2-cyclopentyloxy-3-(1,2,4-triazol-1-yl)-propannitril | 91 |
| 2-(4-Chlorphenyl)-2-cyclopentyloxy-3-(1,2,4-triazol-1-yl)-propannitril, Hydronitrat | 91 |
| <u>Vergleichsmittel</u> | |
| 2-Cyan-2-methoxyiminoessigsäure-äthylaminocarbonylamid | 0 |

Die erfindungsgemäßen Mittel lagen als 10 %ige oder als 20 %ige in Wasser dispergierbare Formulierungen vor.



Formular-Nr.: 1439-8

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1000 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl
Berliner Disconto-Bank AG, Berlin, Konto-Nr. 241/5008, Bankleitzahl 1
Berliner Handels-Gesellschaft – Frankfurter Bank –, Berlin, Konto-Nr. 14-362, Bankleitzahl 100 202 00

SC3 (

PATENTANSPRÜCHE

1. Azolyl-Propannitrile der allgemeinen Formel

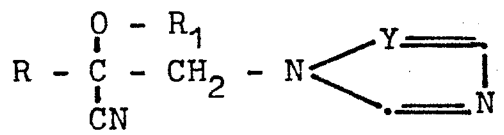

I

in der

R einen gegebenenfalls ein- oder mehrfach, gleich oder verschieden durch Halogen, $(C_1-C_4)$-Alkyl, $(C_1-C_4)$-Alkoxy, Trifluormethyl, Cyano oder die Nitrogruppe substituierten aromatischen Kohlenwasserstoffrest,

$R_1$ einen gegebenenfalls durch $C_1-C_3$-Alkyl substituierten $C_3-C_8$-Cycloalkylrest und

Y N oder CH bedeuten und deren Säureadditionssalze mit anorganischen und organischen Säuren.

2. Azolyl-Propannitrile gemäß Anspruch 1, worin

R 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 3-Jodphenyl, 4-Jodphenyl, 2,4-Dichlorphenyl, 3,4-Dichlorphenyl, 2,6-Dichlorphenyl, 2-Methylphenyl, 3-Methylphenyl, 4-Methylphenyl, 2-Äthylphenyl, 3-Äthylphenyl, 4-Äthylphenyl, 2-Propylphenyl, 3-Propylphenyl, 4-Propylphenyl, 2-Isopropylphenyl, 3-Isopropylphenyl, 4-Isopropylphenyl, 2-Butylphenyl, 3-Butylphenyl, 4-Butylphenyl, 2-sek.-Butylphenyl, 3-sek.-Butylphenyl, 4-sek.-Butylphenyl,



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

2-tert.-Butylphenyl, 3-tert.-Butylphenyl, 4-tert.-Butylphenyl, 2-Methoxyphenyl, 3-Methoxyphenyl, 4-Methoxyphenyl, 2-Äthoxyphenyl, 3-Äthoxyphenyl, 4-Äthoxyphenyl, 2-Methylthiophenyl, 3-Methylthiophenyl, 4-Methylthiophenyl, 2-Äthylthiophenyl, 3-Äthylthiophenyl, 4-Äthylthiophenyl, 2-Trifluormethylphenyl, 3-Trifluormethylphenyl, 4-Trifluormethylphenyl, 2-Nitrophenyl, 3-Nitrophenyl, 4-Nitrophenyl, 3-Fluor-4-methoxyphenyl, 3-Chlor-5-nitrophenyl, 4-Chlor-2-fluorphenyl, 3,4,5-Trimethoxyphenyl oder 5-Chlor-2-nitrophenyl,

$R_1$ Cyclopropyl, Cyclobutyl, Cyclopentyl, Cyclohexyl, Cycloheptyl, Cyclooctyl, 2-Methylcyclopentyl, 2-Methylcyclohexyl, 2,2-Dimethylcyclopentyl oder 2,2-Dimethylcyclohexyl und

Y N oder CH bedeuten.

3. 2-Cyclopentyloxy-3-(imidazol-1-yl)-2-(2-methylphenyl)-propannitril, Hydronitrat
4. 2-Cyclopentyloxy-3-(imidazol-1-yl)-2-(2-methylphenyl)-propannitril
5. 2-Cyclohexyloxy-2-(2-methylphenyl)-3-(1,2,4-triazol-1-yl)-propannitril, Hydronitrat
6. 2-Cyclopentyloxy-2-(2-methylphenyl)-3-(1,2,4-triazol-1-yl)-propannitril, Hydronitrat
7. 2-Cyclohexyloxy-3-(imidazol-1-yl)-2-(2-methylphenyl)-propannitril, Hydronitrat
8. 2-Cyclohexyloxy-3-(imidazol-1-yl)-2-(2-methylphenyl)-propannitril
9. 2-Cyclohexyloxy-2-(2-methylphenyl)-3-(1,2,4-triazol-1-yl)-propannitril

- 55 -

Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 3571

10. 2-Cyclohexyloxy-3-(imidazol-1-yl)-2-phenyl-propannitril
11. 2-Cyclohexyloxy-3-(imidazol-1-yl)-2-phenyl-propannitril, Hydronitrat
12. 2-(2-Chlorphenyl)-2-cyclohexyloxy-3-(imidazol-1-yl)-propannitril
13. 2-(2-Chlorphenyl)-2-cyclohexyloxy-3-(imidazol-1-yl)-propannitril, Hydronitrat
14. 2-Cyclohexyloxy-2-phenyl-3-(1,2,4-triazol-1-yl)-propannitril
15. 2-Cyclohexyloxy-2-phenyl-3-(1,2,4-triazol-1-yl)-propannitril, Hydronitrat
16. 2-(4-Chlorphenyl)-2-cyclohexyloxy-3-(imidazol-1-yl)-propannitril, Hydronitrat
17. 2-(2-Chlorphenyl)-2-cyclohexyloxy-3-(1,2,4-triazol-1-yl)-propannitril
18. 2-(2-Chlorphenyl)-2-cyclohexyloxy-3-(1,2,4-triazol-1-yl)-propannitril, Hydronitrat
19. 2-Cyclopentyloxy-3-(imidazol-1-yl)-2-phenyl-propannitril
20. 2-Cyclopentyloxy-3-(imidazol-1-yl)-2-phenyl-propannitril, Hydronitrat
21. 2-(2-Chlorphenyl)-2-cyclopentyloxy-3-(imidazol-1-yl)-propannitril
22. 2-(2-Chlorphenyl)-2-cyclopentyloxy-3-(imidazol-1-yl)-propannitril, Hydronitrat
23. 2-Cyclopentyloxy-2-phenyl-3-(1,2,4-triazol-1-yl)-propannitril
24. 2-Cyclopentyloxy-2-phenyl-3-(1,2,4-triazol-1-yl)-propannitril, Hydronitrat
25. 2-(2-Chlorphenyl)-2-cyclopentyloxy-3-(1,2,4-triazol-1-yl)-propannitril
26. 2-(2-Chlorphenyl)-2-cyclopentyloxy-3-(1,2,4-triazol-1-yl)-propannitril, Hydronitrat

- 56 -

Formular-Nr.: 1489-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

27. 2-Cyclohexyloxy-2-(2,4-dichlorphenyl)-3-(imidazol-1-yl)-propannitril
28. 2-Cyclohexyloxy-2-(2,4-dichlorphenyl)-3-(imidazol-1-yl)-propannitril, Hydronitrat
29. 2-(4-Chlorphenyl)-2-cyclohexyloxy-3-(1,2,4-triazol-1-yl)-propannitril
30. 2-(4-Chlorphenyl-2-cyclohexyloxy-3-(1,2,4-triazol-1-yl)-propannitril, Hydronitrat
31. 2-Cyclohexyloxy-2-(2,4-dichlorphenyl)-3-(1,2,4-triazol-1-yl)-propannitril
32. 2-Cyclohexyloxy-2-(2,4-dichlorphenyl)-3-(1,2,4-triazol-1-yl)-propannitril, Hydronitrat
33. 2-(4-Chlorphenyl)-2-cyclopentyloxy-3-(imidazol-1-yl)-propannitril
34. 2-(4-Chlorphenyl)-2-cyclopentyloxy-3-(imidazol-1-yl)-propannitril, Hydronitrat
35. 2-(4-Chlorphenyl)-2-cyclopentyloxy-3-(1,2,4-triazol-1-yl)-propannitril
36. 2-(4-Chlorphenyl)-2-cyclopentyloxy-3-(1,2,4-triazol-1-yl)-propannitril, Hydronitrat
37. 2-Cyclopentyloxy-2-(2-fluorphenyl)-3-(imidazol-1-yl)-propannitril
38. 2-Cyclopentyloxy-2-(2-fluorphenyl)-3-(1,2,4-triazol-1-yl)-propannitril
39. 2-Cyclohexyloxy-2-(2-fluorphenyl)-3-(imidazol-1-yl)-pentannitril
40. 2-Cyclohexyloxy-2-(2-fluorphenyl)-3-(1,2,4-triazol-1-yl)-propannitril
41. 2-Cyclopentyloxy-2-(2-fluorphenyl)-3-(imidazol-1-yl)-propannitril
42. 2-Cyclohexyloxy-2-(2-fluorphenyl)-3-(imidazol-1-yl)-propannitril



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 10(
Berliner Disconto-Bank AG, Berlin, Konto-Nr. 241/5008, Bankleitzahl 100 7
Berliner Handels-Gesellschaft — Frankfurter Bank —, Berlin,
Konto-Nr. 14-362, Bankleitzahl 100 202 00

54 FH IV

43. 2-Cyclopentyl-2-(2-methylphenyl)-3-(1,2,4-triazol-1-yl)-propannitril

44. Verfahren zur Herstellung von Azolyl-Propannitrilen gemäß Ansprüchen 1 bis 43, dadurch gekennzeichnet, daß man Verbindungen der allgemeinen Formel

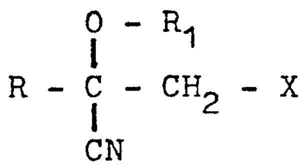

mit Verbindungen der allgemeinen Formel

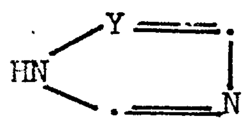

oder deren Alkalisalzen in Gegenwart eines Lösungsmittels und gegebenenfalls in Gegenwart einer Base umsetzt, wobei R, $R_1$ und Y die oben genannte Bedeutung haben und X Halogen oder einen gegebenenfalls in der Seitenkette halogenierten Alkylsulfonyloxy-Rest oder einen Arylsulfonyloxy-Rest darstellt.

45. Biozide Mittel, gekennzeichnet durch einen Gehalt an mindestens einer Verbindung nach den Ansprüchen 1 bis 43.

46. Biozide Mittel gemäß Anspruch 45 mit fungizider Wirkung

47. Biozide Mittel gemäß Anspruch 45 mit wuchsregulatorischer Wirkung.

48. Biozide Mittel gemäß Anspruch 45 mit bakterizider Wirkung.

49. Biozide Mittel gemäß Anspruch 45 in Mischung mit Träger- und/oder Hilfsstoffen.



Formular-Nr.: 1439-2

Vorstand: Dr. Herbert Asmis · Dr. Christian Bruhn · Hans-Jürgen Hamann
Dr. Heinz Hannse · Karl Otto Mittelstenscheid · Dr. Horst Witzel
Vorsitzender des Aufsichtsrats: Dr. Eduard v. Schwartzkoppen
Sitz der Gesellschaft: Berlin und Bergkamen
Handelsregister: AG Charlottenburg 93 HRB 283 u. AG Kamen HRB 0061

Postanschrift: SCHERING AG · D-1 Berlin 65 · Postfach 65 03 11
Postscheck-Konto: Berlin-West 1175-101, Bankleitzahl 100 100 10
Berliner Commerzbank AG, Berlin, Konto-Nr. 108 7006 00, Bankleitzahl 100 400 00
Berliner Disconto-Bank AG, Berlin, Konto-Nr. 241/5008, Bankleitzahl 100 700 00
Berliner Handels-Gesellschaft – Frankfurter Bank –, Berlin,
Konto-Nr. 14-362, Bankleitzahl 100 202 00

54 FH IV 35718

Europäisches Patentamt

**EUROPÄISCHER RECHERCHENBERICHT**

0134515
Nummer der Anmeldung

EP 84108384.3

| Kategorie | EINSCHLÄGIGE DOKUMENTE: Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | DE - A1 - 2 734 365 (ICI)<br>* Anspruch 1 *<br>-- | 1,45 | C 07 D 249/08<br>C 07 D 233/61<br>A 01 N 43/50<br>A 01 N 43/653 |
| A | US - A - 4 366 165 (MILLER)<br>* Tabelle I *<br>-- | 45 | |
| A | EP - A1 - 0 069 448 (ICI)<br>* Beispiel 1 *<br>-- | 1,45 | |
| A | DE - A1 - 2 821 971 (ROHM)<br>* Formel I *<br>-- | 1,45 | |
| A | CHEMICAL ABSTRACTS, Vol. 91, No. 7, 13. August 1979, Columbus, Ohio, USA<br>ANON "Combatting plant fungi" Seite 191, Spalte 2, Abstract-No. 50 957s<br>& Res. Discl. 1979, 181, 231<br>---- | 1 | RECHERCHIERTE SACHGEBIETE (Int. Cl.4)<br>C 07 D 249/00<br>C 07 D 233/00 |

Der vorliegende Recherchenbericht wurde für alle Patentansprüche erstellt.

| Recherchenort | Abschlußdatum der Recherche | Prüfer |
|---|---|---|
| WIEN | 12-10-1984 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument